# EUROPEAN PATENT APPLICATION

(11) **EP 0 716 846 A1**
(43) Date of publication of application: **19.06.1996**
(21) Application number: 95108786.5
(22) Date of filing: 07.06.1995
(51) Int. Cl.: A61K 7/13

(54) **Oxidation hair dye composition**

(30) Priority: 16.12.1994 JP 313175/94
(71) Applicant: Yamahatsu Sangyo Kaisha Ltd., Osaka-shi Osaka-fu (JP)
(72) Inventor: Tsujino, Yoshio, Izumisano-shi, Osaka-fu (JP); Tomura, Kazuyo, Osaka-shi, Osaka-fu (JP)
(74) Representative: VOSSIUS & PARTNER

(57) **Abstract**

A one-pack type oxidation hair dye composition which comprises uricase, an oxidation dye, uric acid and optionally a reducing agent the electrode potential of which is more positive than that of ascorbic acid but more negative than that of uric acid, pH of said composition being 6.7 to 9.5 is disclosed.

## Description

The present invention relates to a one-pack type oxidation hair dye composition using uricase. More particularly, it relates to an oxidation hair dye composition containing uricase stably and having excellent hair dyeing effect.

In conventional oxidation hair dye compositions, hydrogen peroxide, sodium perborate, sodium percarbonate and the like are used as oxidizing agents for developing color by oxidation polymerization of the dyes and for bleaching hair.

However, such oxidizing agents significantly damage hair due to their strong oxidative effect. As a result, they are apt to cause serious cosmetological problems such as split hair, snap hair, unkempt hair and the like. In addition, since it is likely that such oxidizing agents come into contact with skin such as face, hands and the like during treatment with them, they are also apt to cause skin disorder for some persons.

As a means for solving such problems by aiming at mild oxidation by an enzyme, the present inventors have proposed a hair dye composition comprising a dielectron reducing oxidase utilizing oxygen as an acceptor and a substrate as donor, which utilizes the oxidation of an oxidation dye by the enzymatic oxidation reaction for hair dyeing (U.S. Patent 4,961,925).

The present inventors have further studied uricase of this type of the hair dye composition as disclosed in U.S. Patent 4,961,925, intensively, and have succeeded in obtaining a hair dye composition having improved stability of uricase which is quite practicable by selecting a suitable combination of certain ingredients.

The main object of the present invention is to improve stability of uricase in a hair dye composition comprising a dielectron reducing oxidase utilizing oxygen as an acceptor and a substrate as donor, which utilizes the oxidation of an oxidation dye by the enzymatic oxidation reaction for hair dyeing to increase practicability of such type of the hair dye composition.

This object as well as other objects and advantages of the present invention will become apparent to those skilled in the art from the following description.

According to the present invention, there is provided a one-pack type oxidation hair dye composition which comprises uricase, an oxidation dye, uric acid and optionally a reducing agent the electrode potential of which is more positive than that of ascorbic acid but more negative than that of uric acid, pH of said composition being 6.7 to 9.5.

Uricase contained in the oxidation hair dye composition of the present invention is also referred to as "urate oxidase" and is a dielectron reducing oxidase which is contained in liver, kidney, spleen and the like of mammal other than primates in a large amount.

The concentration of the uricase to be contained is 1 to 1,000 I.U./g, preferably, 100 to 250 I.U./g based on the weight of the composition. When the concentration is less than 1 I.U./g, hair dyeing effect is insufficient. On the other hand, when it is more than 1,000 I.U./g, increase in hair dyeing effect is not expected any more and it is disadvantageous from economicl viewpoint.

The term "I.U." as the unit of enzyme activity used herein means the international unit and is the amount of enzyme activity required for converting 1 µmole of uric acid into its reaction product per one minute under conditions of pH 8.5 at 25°C.

Uric acid which is the substrate of uricase is also an essential ingredient of the present invention. Then, upon using the oxidation hair dye composition of the present invention, uricase reacts with the substrate uric acid as donor, under suitable conditions to polymerize the oxidation dye by its enzymatic oxidation reaction in hair to form a pigment and to dye the hair. The term "uric acid" used herein includes salts thereof.

The concentration of uric acid to be contained in the oxidation hair dye composition is preferably 0.1 to 5.0% by weight. Like the above description with respect to the enzyme, when the concentration of uric acid as the donor is less than the above range, the hair dyeing effect is insufficient. On the other hand, when the concentration is more than the above range, any increase in the hair dyeing effect is not expected and it is disadvantageous from the economical viewpoint.

In the composition of the present invention, preferably, a certain reducing agent is contained. The reducing agent is necessary to have a suitable anti-oxidant activity without inhibiting the hair dyeing effect by the enzymatic enzyme reaction. Then, the electrode potential of the reducing agent is more positive than that of ascorbic acid but more negative than that of uric acid. Such a reducing agent has the electrode potential of about -80 mV to about 68 mV as measured by the method as described hereinafter. Examples of the reducing agent include dl-cysteine, thioglycolic acid, N-acetyl-L-cysteine, sodium sulfite and salts thereof.

The concentration of the reducing agent to be contained in the oxidation hair dye composition of the present invention is preferably not more than 0.5% by weight based on the total weight of the composition.

In the present invention, the desired composition can be obtained without using any reducing agent, when it is produced under strict anaerobic conditions. However, normally, it is preferred to use the reducing agent in view of ease of the production.

The oxidation hair dye composition of the present invention is adjusted to pH 6.7 to 9.5. It has been found that uricase is stabilized by this pH adjustment and excellent hair dyeing effect can be obtained.

The pH adjusting agent which can be used in the present invention includes potassium hydroxide and monoethanolamine and they can be used alone or in combination thereof.

The oxidation dye to be used in the present invention and its amount are not specifically limited. For example, the oxidation dye described in Quasi-drug Material Specification (supervised by Ministry of Health and Welfare, Pharmaceutical and Supply Bureau, Japan, edited by Japan Official Compendium Society, published by Yakuji Nippo Co., Ltd., June, 1991) can be used in a suitable amount. Examples of the dyes include 5-amino-o-cresol, 3,3'-iminodiphenol, 2,4-diaminophenol hydrochloride, toluene-2,5-diamine hydrochloride, p-phenylenediamine hydrochloride, N-phenyl-p-phenylenediamine hydrochloride, m-phenylenediamine hydrochloride, o-aminophenol, catechol, N-phenyl-p-phenylenediamine acetate, 2,6-diaminopyridine, 1,5-dihydroxynaphthalene, diphenylamine, toluene-2,5-diamine, toluene-3,4-diamine, α-naphthol, p-aminophenol, p-phenylenediamine, p-methylaminophenol, hydroquinone, pyrogallol, N-phenyl-p-phenylenediamine, phloroglucin, m-aminophenol, m-phenylenediamine, 5-amino-o-cresol sulfate, o-aminophenol sulfate, o-chloro-p-phenylenediamine sulfate, 4,4-diaminodiphenylamine sulfate, 2,4-diaminophenol sulfate, toluene-2,5-diamine sulfate, p-aminophenol sulfate, p-phenylenediamine sulfate, p-methylaminophenol sulfate, m-aminophenol sulfate, m-phenylenediamine sulfate, 2,4-diaminophenoxyethanol hydrochloride and 5-(2-hydroxyethylamino)-2-methylphenol.

Direct dyes which can be often used in combination with oxidation dyes are also included in the oxidation dyes in a wide sense and can be used in the present invention. Examples thereof include 2-amino-4-nitrophenol, 2-amino-5-nitrophenol, 1-amino-4-methylaminoanthraquinone, nitro-p-phenylenediamine hydrochloride, 1,4-diaminoanthraquinone, nitro-p-phenylenediamine, picramic acid, sodium picramate, 2-amino-5-nitrophenol sulfate, resorcinol, nitro-p-phenylenediamine sulfate, p-nitro-o-phenylenediamine sulfate, p-nitro-m-phenylenediamine sulfate and the like.

Among them, particularly preferred oxidation dyes used in the composition of the present invention are p-phenylenediamine and salts thereof, p-aminophenol, 5-amino-o-cresol, p-methyl-p-aminophenol sulfate, m-aminophenol, p-nitro-o-phenylenediamine, 2,6-diaminopyridine, resorcinol, o-aminophenol, and m-phenylenediamine hydrochloride.

These hair dyes can be used alone or in combination of two or more thereof.

For improving hair dyeing effect, in the oxidation hair dye composition of the present invention, it is necessary to allow an electron to move freely upon oxidation polymerization of the dye. For this, preferably, the composition should contain at least 60% by weight, more preferably, 60 to 99% by weight of water.

In addition to the above ingredients, the oxidation hair dye composition of the present invention can contain other ingredients such as surfactants, oil ingredients, thickeners, solvents, chelating agents, perfumes and the like. It has also been found that xanthan gum, particularly, that free from any proteolytic enzyme and metal salt is suitable for dispersing uric acid. In the present invention, it is preferred to use such a xanthan gum as an ingredient for dispersing uric acid.

The following experiments further illustrate the pH range suitable for the oxidation hair dye composition of the present invention, the reducing agent and the pH adjusting agent to be contained in the composition and the amount of water in the composition.

### Experiment 1

### Relationship between pH of composition and stability of enzyme and hair dyeing effect

Hair dye compositions to be tested which had been adjusted to various pH values under nitrogen atmosphere (anaerobic conditions) were stored at 40°C for 20 days. After storage, enzymatic activities, pH and hair dyeing effect of the compositions were measured. Then, the suitable pH range was determined from the view point of storage stability of the enzyme activity and hair dyeing effect.

### Method

**Table 1**

| Ingredients | Amount (% by weight) |
|---|---|
| p-Phenylenediamine | 1.5 |
| p-Aminophenol | 0.1 |
| m-Aminophenol | 0.08 |
| m-Phenylenediamine hydrochloride | 0.12 |
| Glycine | 0.8 |
| Sodium sulfite | 0.126 |
| Glycerol | 3.0 |
| Uric acid | 1.0 |
| Xanthan gum | 1.0 |
| Sodium carboxymethylcellulose | 1.0 |
| Uricase (22 I.U./mg) | 0.4545 |
| 4N Aqueous potassium hydroxide solution | suitable amount |
| Purified Water | suitable amount |
| 0.05M Aqueous phosphate solution | suitable amount |

The ingredients shown in Table 1 were mixed in a box the atmosphere in which had been replaced with nitrogen beforehand and the pH thereof was suitably adjusted. The resultant composition and that filled in a 20 g aluminum tube and stored at 40°C for 20 days were used as test samples and they were subjected to the following tests.

### Evaluation of time-course stability of uricase activity

Activity assay for uricase was measured by using an oxygen electrode (Clark Type) system. About 1 g of each test sample was dissolved into 20 g of purified water, while replacing the atmosphere with nitrogen. Then, 0.3 ml of the solution was taken in a reaction cell dissolved oxygen measuring apparatus containing 3 ml of a bulk solution (1mM uric acid/50mM borate-KOH buffer, (pH 8.5)) at 25°C. The rate of decrease in dissolved oxygen caused by the enzymatic reaction was measured and the residual enzyme activity was evaluated by a relative value obtained by taking the enzyme activity immediately after the production of the composition as 100.

### Measurement of pH value

The pH value was measured after diluting the test samples ten times with purified water.

### Hair dyeing test

The test sample was applied on a goat hair in a bath volume ratio of the test sample to the goat hair of 1 : 2 and the goat hair was incubated under 90% relative humidity at 30°C for 30 minutes. Then, it was washed with water, dried, and evaluated the hair dyeing effect by using JIS Standard Color Chips.

The results are shown in Table 2.

**Table 2**

| Sample No. | Buffer solution | | pH | Residual enzyme activity* (after 20 days storage) | Hair dyeing effect** | |
|---|---|---|---|---|---|---|
| | 4N KOH (ml) | H₃PO₄ | | | immediately after production | after 20 days storage |
| 1-1 | - | balance | 6.3 | 73.5 | 8.00 | 8.00 |
| 1-2 | - | - | 6.7 | 90.2 | 4.00 | 4.00 |
| 1-3 | 1.0 | - | 7.4 | 115.8 | 3.00 | 4.00 |
| 1-4 | 2.2 | - | 8.8 | 100.6 | 3.00 | 3.50 |
| 1-5 | 2.2 | - | 9.0 | 99.3 | 3.00 | 3.00 |
| 1-6 | 3.0 | - | 9.5 | 92.0 | 3.00 | 4.00 |
| 1-7 | 4.0 | - | 9.9 | 73.0 | 3.00 | 4.00 |
| 1-8 | 4.5 | - | 10.2 | 35.8 | 3.00 | 5.00 |

| | | | | | | |
|---|---|---|---|---|---|---|
| *: The value was calculated by taking the activity immediately after the production as 100. | | | | | | |
| **: Evaluated by using JIS Standard Color Chips. | | | | | | |

As seen from Table 2, it has been found that both enzyme activity and hair dyeing effect are stable in the pH range of 6.7 to 9.5, while both properties are remarkably deteriorated when the pH deviates from this pH range.

Thus, the pH range of the hair dye composition of the present invention should be within the range of pH 6.7 to 9.5.

### Experiment 2

### Evaluation of reducing agents

Various reducing agents generally used as raw materials of cosmetics were tested for their influence on hair dyeing effect. At the same time, the electrode potentials of those reducing agents were measured. Thereby, reducing agents suitable for the composition of the present invention were selected and their suitable electrode potentials were determined.

### (1) Influence of reducing agents on hair dyeing effect

### Method

To 5 g of each hair dye composition base as shown in Table 3-1 were added 0.5 ml of an enzyme solution (2,000 I.U./ml) and 0.05 g of uric acid and the mixture was thoroughly mixed. The resultant composition was immediately applied on a goat hair weighed 2 g and of 10 cm long. Then, the goat hair was incubated under 90% relative humidity at 30°C for 30 minutes, washed with water and shampooed, dried and observed the dyeing properties. The hair dyeing effect of the treated goat hair was evaluated in visual according to the following criteria.
A: being dyed in a thick dark brown color
B: being dyed in a dark brown color
C: being dyed in a pale dark brown color
X: being hardly dyed

### (2) Measurement of electrode potentials of reducing agents

Electrode potentials of the reducing agents used in the above (1) were measured and the range of the electrode potential of the reducing agent to be used in the composition of the present invention was determined.

### Method

Apart from the dye composition base as shown in Table 3-1, the various reducing agents as shown in Table 3-2 were adjusted to the final concentration of 500 µM, while replacing the atmosphere with nitrogen and their electrode potentials were measured by potentiometric titration.

The results of Experiments (1) and (2) are shown in Tables 3-1 and 3-2 together with the experimental conditions.

**Table 3-1**

| Ingredients | Amounts (% by weight) in dye composition base | | | | | | |
|---|---|---|---|---|---|---|---|
| | 2-1 | 2-2 | 2-3 | 2-4 | 2-5 | 2-6 | 2-7 |
| p-Phenylene diamine | 1.0 | 1.0 | 1.0 | 1.0 | 1.0 | 1.0 | 1.0 |
| m-Phenylene diamine | 0.5 | 0.5 | 0.5 | 0.5 | 0.5 | 0.5 | 0.5 |
| m-Aminophenol | 0.05 | 0.05 | 0.05 | 0.05 | 0.05 | 0.05 | 0.05 |
| Glycerol | 3.0 | 3.0 | 3.0 | 3.0 | 3.0 | 3.0 | 3.0 |
| Sodium carboxymethylcellulose | 1.0 | 1.0 | 1.0 | 1.0 | 1.0 | 1.0 | 1.0 |
| Uric acid | - | 15mM | - | - | - | - | - |
| Sodium sulfite | - | - | 15mM | - | - | - | - |
| Thioglycolic acid | - | - | - | 15mM | - | - | - |
| N-Acetyl-L-cysteine | - | - | - | - | 15mM | - | - |
| Sodium ascorbate | - | - | - | - | - | 15mM | - |
| dl-Cysteine | - | - | - | - | - | - | 15mM |
| 4N KOH | to adjust to pH 9 | | | | | | |
| Purified water | 100 g balance | | | | | | |
| Hair dyeing effect | A | A | A | A | A | X | B |

**Table 3-2**

| Reducing agents | Uric acid | Na₂SO₃ | Thioglycolic acid | N-acetyl-L-cysteine | Na ascorbate | dl-cysteine |
|---|---|---|---|---|---|---|
| Electrode potential (mV)* (Pt/AgCl) | 68 | 35 | -21 | 15 | -82 | -66 |

### Conditions for measurement

Buffer solution: 0.5M glycine-potassium hydroxide buffer solution (pH 8.5)
Standard solution: 0.01N iodine solution
Working electrode: Platinum
Reference electrode: Ag/AgCl
Apparatus: Hiranuma Reporting Titrator, Comtite 101
As seen from the results of Table 3, uric acid, sodium sulfite, thioglycolic acid, N-acetyl-L-cysteine and dl-cysteine showed good hair dyeing effect, while the effect of sodium ascorbate was insufficient. In addition, in view of the fact that the uric acid which is the essential ingredient of the present invention acts as the substrate of the enzyme, it has been found that, suitably, the electrode potential of the reducing agent to be added to the composition is more positive than that of ascorbic acid but more negative than uric acid, that is, about -80 mV to about 68 mV.

### Experiment 3

### Evaluation of pH adjusting agents

### Method

As shown in Table 4, the hair dyeing effect (dyeing properties) was evaluated with changing a pH adjusting agent (alkaline materials). The compositions (hair dye pastes) were adjusted to pH 8.5 with various pH adjusting agents, filled into sealed vessels and stored at 40°C for 120 days. Then, 4g of each hair dyes paste was taken out from the vessel and immediately applied to the goat hair which weighed 2 g and was 10 cm long. The goat hair was incubated under 90% relative humidity at 30°C for 30 minutes and then washed with water, shampooed and dried. The hair dyeing effect of the treated goat hair was visually evaluated according to the following criteria.
A: being dyed in a thick dark brown color
B: being dyed in a dark brown color
C: being dyed in a pale dark brown color
X: being hardly dyed
The results are shown in Table 4.

**Table 4**

| Ingredients | Amount (% by weight) | | | |
|---|---|---|---|---|
| | 3-1 | 3-2 | 3-3 | 3-4 |
| p-Phenylenediamine | 1.2 | 1.2 | 1.2 | 1.2 |
| m-Phenylenediamine | 0.1 | 0.1 | 0.1 | 0.1 |
| p-Aminophenol | 0.6 | 0.6 | 0.6 | 0.6 |
| Glycerol | 3.0 | 3.0 | 3.0 | 3.0 |
| Sodium carboxymethylcellulose | 1.0 | 1.0 | 1.0 | 1.0 |
| Glycine | 0.8 | 0.8 | 0.8 | 0.8 |
| Decaglyceryl monooleate | 2.0 | 2.0 | 2.0 | 2.0 |
| Thioglycolic acid | 13mM | 13mM | 13mM | 13mM |
| 4N Aqueous potassium hydroxide solution | 2.82 | - | - | - |
| 4N Aqueous sodium hydroxide solution | - | 2.85 | - | - |
| Aqueous ammonia (28%) | - | - | 0.61 | - |
| Monoethanolamine | - | - | - | 0.61 |
| Uric acid | 1.0 | 1.0 | 1.0 | 1.0 |
| Uricase (k I.U.) | 10 | 10 | 10 | 10 |
| Purified water | 100 g balance | | | |
| Hair dyeing effect | A | B | X | A |

As seen from Table 4, excellent hair dyeing effect was obtained only when the pH was adjusted by potassium hydroxide or monoethanolamine.

### Experiment 4

### Relation between amount of water in composition and hair dyeing effect

It was considered that a certain amount of water would be required for the composition of the present invention so that movement of an electron necessary for oxidation polymerization of a dye was assured to improve its hair dyeing effect. Then, hair dyeing effect was evaluated with appropriately changing the amount of water and the amount of water necessary for the oxidation hair dye composition was determined.

### Method

As seen from Tables 5 and 6, various test samples were prepared by replacing purified water with 1,3-butylene glycol or glycerol and 5 g of each sample was applied to 2 g of goat hair and wool cloth. They were incubated under 90% relative humidity at 30°C for 30 minutes and then washed with water and dried.

The hair dyeing effect of the dried goat hair and wool cloth were evaluated by measuring their brightness by using JIS Standard Color Chips.

The results are also shown in Tables 5 and 6.

**Table 5**

| Ingredients | Amount (% by weight) | | | | | |
|---|---|---|---|---|---|---|
| | Standard | 4-1 | 4-2 | 4-3 | 4-4 | 4-5 |
| p-Phenylenediamine | 1.5 | 1.5 | 1.5 | 1.5 | 1.5 | 1.5 |
| p-Aminophenol | 0.1 | 0.1 | 0.1 | 0.1 | 0.1 | 0.1 |
| m-Aminophenol | 0.08 | 0.08 | 0.08 | 0.08 | 0.08 | 0.08 |
| m-Phenylenediamine hydrochloride | 0.12 | 0.12 | 0.12 | 0.12 | 0.12 | 0.12 |
| 4N Aqueous potassium hydroxide solution | 2.62 | 2.62 | 2.62 | 2.62 | 2.62 | 2.62 |
| Uric acid | 1.0 | 1.0 | 1.0 | 1.0 | 1.0 | 1.0 |
| Uricase (24.1 I.U./mg) | 0.83 | 0.83 | 0.83 | 0.83 | 0.83 | 0.83 |
| Glycine | 0.8 | 0.8 | 0.8 | 0.8 | 0.8 | 0.8 |
| Sodium sulfite | 0.126 | 0.126 | 0.126 | 0.126 | 0.126 | 0.126 |
| Glycerol | 3.0 | 3.0 | 3.0 | 3.0 | 3.0 | 3.0 |
| Sodium carboxymethylcellulose | 1.0 | 1.0 | 1.0 | 1.0 | 1.0 | 1.0 |
| Xanthan gum | 1.0 | 1.0 | 1.0 | 1.0 | 1.0 | 1.0 |
| Decaglyceryl monooleate | 3.0 | 3.0 | 3.0 | 3.0 | 3.0 | 3.0 |
| 1,3-Butylene glycol | 0 | 8.48 | 16.96 | 25.45 | 33.93 | 42.41 |
| Purified water | 84.82 | 76.34 | 67.86 | 59.37 | 50.89 | 42.41 |

| Hair dyeing effect* | | | | | | |
|---|---|---|---|---|---|---|
| Goat hair bundle | 3.25 | 3.75 | 4.25 | 6.25 | 7.00 | 7.50 |
| Wool cloth | 3.00 | 3.25 | 3.75 | 4.00 | 4.50 | 4.25 |

| | | | | | | |
|---|---|---|---|---|---|---|
| *: Value of JIS Color Standard Chips | | | | | | |

**Table 6**

| Ingredients | Amount (% by weight) | | | | | |
|---|---|---|---|---|---|---|
| | Standard | 5-1 | 5-2 | 5-3 | 5-4 | 5-5 |
| p-Phenylenediamine | 1.5 | 1.5 | 1.5 | 1.5 | 1.5 | 1.5 |
| p-Aminophenol | 0.1 | 0.1 | 0.1 | 0.1 | 0.1 | 0.1 |
| m-Aminophenol | 0.08 | 0.08 | 0.08 | 0.08 | 0.08 | 0.08 |
| m-Phenylenediamine hydrochloride | 0.12 | 0.12 | 0.12 | 0.12 | 0.12 | 0.12 |
| 4N Aqueous potassium hydroxide solution | 2.62 | 2.62 | 2.62 | 2.62 | 2.62 | 2.62 |
| Uric acid | 1.0 | 1.0 | 1.0 | 1.0 | 1.0 | 1.0 |
| Uricase (20 I.U./mg) | 1.0 | 1.0 | 1.0 | 1.0 | 1.0 | 1.0 |
| Glycine | 0.8 | 0.8 | 0.8 | 0.8 | 0.8 | 0.8 |
| Sodium sulfite | 0.126 | 0.126 | 0.126 | 0.126 | 0.126 | 0.126 |
| Sodium carboxymethylcellulose | 1.0 | 1.0 | 1.0 | 1.0 | 1.0 | 1.0 |
| Xanthan gum | 1.0 | 1.0 | 1.0 | 1.0 | 1.0 | 1.0 |
| Decaglyceryl monooleate | 3.0 | 3.0 | 3.0 | 3.0 | 3.0 | 3.0 |
| Glycerol | 3.0 | 11.46 | 19.93 | 28.39 | 36.86 | 45.32 |
| Purified water | 84.65 | 76.19 | 67.72 | 59.26 | 50.79 | 42.33 |

| Hair dyeing effect* | | | | | | |
|---|---|---|---|---|---|---|
| Goat hair bundle | 2.50 | 2.50 | 3.00 | 4.50 | 6.50 | 7.00 |
| Wool cloth | 2.50 | 2.50 | 2.50 | 3.50 | 4.00 | 5.50 |

| | | | | | | |
|---|---|---|---|---|---|---|
| *: Value of JIS Color Standard Chips | | | | | | |

As seen from Tables 5 and 6, the hair dyeing effect is remarkably decreased when the amount of water becomes less than 60% by weight based on the total weight of the composition in the case that purified water is replaced with 1,3-butylene glycol or glycerol. Therefore, the amount of water to be contained in the oxidation hair dye composition of the present invention is preferably at least 60% by weight based on the total weight of the composition.

Hereinafter, the production of the oxidation hair dye composition of the present invention will be explained.

The hair dye composition of the present invention is produced as a one-pack type preparation in the form of liquid, paste, gel, cream, aerosol or the like according to a conventional method. In view of ease of application and treatment, an aerosol is preferred.

For example, the composition can be produced as follows. The ingredients other than uric acid, uricase, the reducing agent, purified water and the pH adjusting agent are dissolved in a portion of purified water with heating at about 60°C under nitrogen atmosphere. After mixing the mixture for a while to obtain a uniform mixture, the resultant mixture is cooled to about 30°C. To the cooled mixture are added uric acid and the reducing agent suspended in a portion of purified water. Then, pH of the mixture is adjusted to pH 6.7 to 9.5 with the pH adjusting agent. Finally, uricase dissolved in the remaining purified water is added to obtain the desired composition of the present invention.

Alternatively, the composition of the present invention can be produced by the same manner even without any reducing agent when the production is carried out under strict inert gas atmosphere such as nitrogen atmosphere.

The oxidation hair dye composition of the present invention thus obtained is in the form of a one-pack type composition which can be used conveniently and stored in a sealed vessel. When it is used, the composition can be suitably applied as it is to dye the human white hair.

The following Examples further illustrate the present invention in detail but are not to be construed to limit the scope thereof.

### Example 1

The ingredients as shown in the following formulation except uric acid, uricase and the pH adjusting agent were mixed and dissolved in a portion of purified water at 60°C under reduced pressure and the atmosphere was replaced with nitrogen. The mixture was mixed for a while to obtain a uniform mixture. Then, the mixture was cooled to 30°C and to this was added uric acid dispersed in a potion of purified water. The mixture was adjusted to pH 9.0 with potassium hydroxide. Finally, uricase dissolved in the remaining purified water was added to obtain the desired hair dye composition of the present invention in the form of gel.

### Formulation

| Ingredients | Amount (% by weight) |
|---|---|
| p-Phenylenediamine | 0.6 |
| p-Aminophenol | 0.2 |
| m-Aminophenol | 0.1 |
| Polyoxyethylene (4.2) lauryl ether | 3.0 |
| Xanthan gum | 2.0 |
| Uricase (20 I.U./mg) | 0.6 |
| Potassium urate | 2.2 |
| Thioglycolic acid | 0.2 |
| Potassium hydroxide, Purified water (adjusted to pH 9.0 with potassium hydroxide) | balance |

When this composition was applied to human white hair and the white hair was treated at 30°C for 30 minutes, washed with water, shampooed, and then dried, the white hair was dyed in a grayish color.

### Example 2

According to the same manner as described in Example 1, the desired hair cream type composition or the present invention was produced by using the ingredients as shown in the following formulation and adjusting to pH 8.5 with monoethanolamine.

### Formulation

| Ingredients | Amount (% by weight) |
|---|---|
| p-Phenylenediamine | 0.1 |
| m-Phenylenediamine hydrochloride | 0.5 |
| 1,3-Butylene | 2.0 |
| Decaglyceryl monostearate | 3.0 |
| Cetyl alcohol | 0.5 |
| Stearic acid | 0.8 |
| Xanthan gum | 1.0 |
| Sodium carboxymethylcellulose | 1.0 |
| Uric acid | 1.0 |
| Sodium sulfite | 0.08 |
| Uricase (20 I.U./mg) | 1.0 |
| Monoethanolamine, Purified water (adjusted to pH 8.5 with monoethanolamine) | balance |

This composition was applied to human white hair and the white hair was daily treated for 10 to 30 minutes, washed with water, shampooed, and then dried. After treatment for 10 days, the white hair was dyed in a purplish color.

### Example 3

According to the same manner as described in Example 1, the desired hair cream type hair dye composition of the present invention was produced by using the ingredients as shown in the following formulation and adjusting to pH 8.75 with monoethanolamine.

### Formulation

| Ingredients | Amount (% by weight) |
|---|---|
| p-Phenylenediamine | 2.0 |
| m-Phenylenediamine hydrochloride | 0.1 |
| m-Aminophenol | 0.8 |
| Sodium sulfite | 0.08 |
| Polyoxyethylene (10) cetyl ether | 8.0 |
| Stearyl alcohol | 2.5 |
| Oleyl alcohol | 5.0 |
| Behenyl alcohol | 2.0 |
| Cetyl alcohol | 2.0 |
| Cetyltrimethyl ammonium chloride | 1.0 |
| Glycerol | 2.0 |
| Uricase (20 I.U./mg) | 1.5 |
| Uric acid | 5.0 |
| Monoethanolamine, Purified water (adjusted to pH 8.75 with monoethanolamine) | balance |

When this composition was applied to human white hair and the white hair was treated at 30°C for 30 minutes, washed with water, shampooed, and then dried, the white hair was dyed in a grayish color.

### Example 4

According to the same manner as described in Example 1, the desired treatment type hair dye composition of the present invention was produced by using the ingredients as shown in the following formulation and adjusting to pH 8.5 with potassium hydroxide.

### Formulation

| Ingredients | Amount (% by weight) |
|---|---|
| p-Phenylenediamine | 0.04 |
| p-Aminophenol | 0.02 |
| o-Aminophenol | 0.2 |
| Cetyltrimethylammonium chloride | 2.5 |
| Stearyltrimethylammonium chloride | 1.0 |
| Isopropyl myristate | 7.0 |
| Cetyl alcohol | 5.0 |
| Stearyl alcohol | 2.0 |
| Liquid paraffin | 4.0 |
| Liquid lanolin | 0.5 |
| Propylene glycol | 0.5 |
| Uricase (20 I.U./mg) | 1.0 |
| Uric acid | 2.0 |
| Thioglycolic acid | 0.06 |
| Potassium hydroxide, Purified water (adjusted to pH 8.5 with potassium hydroxide) | balance |

When this composition was applied to human white hair and the white hair was treated for 30 minutes, washed with water, shampooed, and then dried, the white hair was dyed in a brownish color.

In addition, when the composition was applied to human white hair after washing with a shampoo and the hair was treated at 30 to 40°C for 5 to 10 minutes as conventional hair treatment several days, after treatment for 10 days, the hair was dyed in a reddish color.

### Example 5

According to the same manner as described in Example 1, the desired gel type hair dye composition of the present invention was produced by using the ingredients as shown in the following formulation without any reducing agent and adjusting to pH 8.5 with potassium hydroxide.

### Formulation

| Ingredients | Amount (% by weight) |
|---|---|
| p-Phenylenediamine | 0.6 |
| p-Aminophenol | 0.2 |
| m-Aminophenol | 0.1 |
| Polyoxyethylene (4.2) lauryl ether | 3.0 |
| Xanthan gum | 2.0 |
| Uricase (20 I.U./mg) | 0.6 |
| Potassium urate | 2.2 |
| Potassium hydroxide, Purified water (adjusted to pH 9.0 with potassium hydroxide) | balance |

When this composition was applied to human white hair and the white hair was treated at 30°C for 30 minutes, washed with water, shampooed, and then dried, the white hair was dyed in a grayish color.

### Example 6

According to the same manner as described in Example 1, the desired hair gel type hair dye composition of the present invention was produced by using the ingredients as shown in the following formulation without any reducing agent and adjusting to pH 8.5 with monoethanolamine.

### Formulation

| Ingredients | Amount (% by weight) |
|---|---|
| p-Phenylenediamine | 0.1 |
| m-Phenylenediamine hydrochloride | 0.5 |
| Propylene glycol | 1.0 |
| Glycine | 0.8 |
| Xanthan gum | 2.0 |
| 2-Alkyl-N-carboxymethyl-N-hydroxyethylimidazolinium betaine | 2.5 |
| Uric acid | 1.0 |
| Uricase (20 I.U./mg) | 1.0 |
| Disodium editate | 0.2 |
| Monoethanolamine, Purified water (adjusted to pH 8.5 with monoethanolamine) | balance |

This composition was applied to human white hair and the white hair was daily treated for 10 to 30 minutes, washed with water, shampooed, and then dried. After treatment for 10 days, the white hair was dyed in a purplish color.

### Example 7

The desired aerosol type hair dye composition of the present invention was produced by using the ingredients as shown in the following formulation, adjusting to pH 8.7 with potassium hydroxide under nitrogen atmosphere and filling the resultant mixture in a aerosol can together with about 4% by weight of liquid propane gas (5.1 kg/cm²) based on the total weight of the entire composition in the can.

### Formulation

| Ingredients | Amount (% by weight) |
|---|---|
| p-phenylenediamine | 1.5 |
| p-Aminophenol | 0.1 |
| m-Aminophenol | 0.12 |
| m-Phenylenediamine hydrochloride | 0.08 |
| Glycine | 0.8 |
| Decaglyceryl monooleate | 3.0 |
| Uric acid | 1.0 |
| Uricase (20 I.U./mg) | 1.0 |
| Potassium hydroxide, Purified water (adjusted to pH 8.7 with potassium hydroxide) | balance |

When this composition was applied to human white hair and the white hair was treated at 30°C for 30 minutes, washed with water, shampooed, and then dried, the white hair was dyed in a grayish color.

### Example 8

According to the same manner as described in Example 1, the desired hair gel type composition or the present invention was produced by using the ingredients as shown in the following formulation and adjusting to pH 8.5 with monoethanolamine.

### Formulation

| Ingredients | Amount (% by weight) |
|---|---|
| p-Phenylenediamine | 0.1 |
| m-Phenylenediamine hydrochloride | 0.5 |
| Propylene glycol | 1.0 |
| Glycine | 0.8 |
| Xanthan gum | 2.0 |
| 2-Alkyl-N-carboxymethyl-hydroxyethylimidazolinium betaine | 2.5 |
| Uric acid | 1.0 |
| Sodium sulfite | 0.08 |
| Uricase (20 I.U./mg) | 1.0 |
| Monoethanolamine, Purified water (adjusted to pH 8.5 with monoethanolamine) | balance |

This composition was applied to human white hair and the white hair was daily treated for 10 to 30 minutes, washed with water, shampooed, and then dried. After treatment for 10 days, the white hair was dyed in a purplish color.

### Example 9

The desired aerosol type hair dye composition of the present invention was produced by using the ingredients as shown in the following formulation, adjusting to pH 8.7 with potassium hydroxide under nitrogen atmosphere and filling the resultant mixture in a aerosol can together with about 4% by weight of liquid propane gas (5.1 kg/cm²) based on the total weight of the entire composition in the can.

### Formulation

| Ingredients | Amount (% by weight) |
|---|---|
| p-Phenylenediamine | 1.5 |
| p-Aminophenol | 0.1 |
| m-Aminophenol | 0.12 |
| m-Phenylenediamine hydrochloride | 0.08 |
| Glycine | 0.8 |
| Glycerol | 1.0 |
| Sodium sulfite | 0.12 |
| Xanthan gum | 0.6 |
| Decaglyceryl monooleate | 1.0 |
| Uric acid | 0.6 |
| Uricase (20 I.U./mg) | 0.8 |
| Potassium hydroxide, Purified water (adjusted to pH 8.7 with potassium hydroxide) | balance |

When this composition was applied to human white hair and the white hair was treated at 30°C for 30 minutes, washed with water, shampooed, and then dried, the white hair was dyed in a grayish color.

### Experiment 5

### Time-course evaluation of stability of hair dyeing effect and uricase activity

| Ingredients | Amount (% by weight) |
|---|---|
| p-Phenylenediamine | 1.5 |
| p-Aminophenol | 0.1 |
| m-Aminophenol | 0.08 |
| m-Phenylenediamine hydrochloride | 0.12 |
| Glycine | 0.8 |
| Glycerol | 3.0 |
| Xanthan gum | 1.0 |
| Sodium carboxymethylcellulose | 1.0 |
| Sodium sulfite | 0.13 |
| Decaglyceryl monooleate | 3.0 |
| Uric acid | 1.0 |
| Uricase (20 I.U./mg) | 1.0 |
| Potassium hydroxide, Purified water (adjusted to pH 8.7 with potassium hydroxide) | balance |

According to the same manner as described in Example 1, oxidation hair dye compositions were produced by using the above ingredients. Then, the compositions were filled in sealed vessels under inert gas atmosphere, stored at 40°C for 2, 4 or 6 months and their hair dyeing effect and uricase activities were evaluated.

### (1) Evaluation of time-course stability of hair dyeing effect

According to the same manner as described in Experiment 2, the hair dyeing effect of each composition was evaluated based on the following criteria.
A: being dyed in a thick gray color
B: being dyed in a gray color
C: being dyed in a pale gray color
X: being hardly dyed

### (2) Evaluation of time-course stability of dyeing properties

According to the same manner as the test for hair dyeing effect, white cloth (JIS L0803 attached white cloth for Color Fastness Test, reference number 1 wool) was dyed with the composition under 75% relative humidity at 30°C for 30 minutes and brightness was measured by using JIS Standard Color Chips to evaluate dyeing properties and the dyeing effect.

### (3) Evaluation of time-course stability of uricase activity

According to the same manner as described in Experiment 1, the enzyme activity was measured,

The results are shown in Table 7.

**Table 7**

| | Initiation | Storage period (months) | | |
|---|---|---|---|---|
| | | 2 | 4 | 6 |
| Hair dyeing effect | A | A | A | A |
| Dyeing properties | 4.25 | 4.25 | 4.25 | 4.50 |
| Residual enzyme activity* | 100.0 | 103.6 | 100.3 | 99.1 |

| | | | | |
|---|---|---|---|---|
| *: The relative value obtained by taking the enzyme activity immediately after the production as 100. | | | | |

As seen from Table 7, according to the present invention, the uricase activity and the hair dyeing effect based on the enzyme activity are stabilized for a long period of time.

As described herein above, according to the present invention, there is provided an oxidation hair dye composition which contains urease stably and exhibits excellent hair dyeing effect.

## Claims

1. A one-pack type oxidation hair dye composition which comprises uricase, an oxidation dye and uric acid, whereby the pH of said composition is 6.7 to 9.5.

2. The oxidation hair dye composition according to claim 1, wherein the composition contains about 0.1 to 5.0% by weight of oxidizing agent and about 0.1 to 5.0% by weight of uric acid.

3. The oxidation hair dye composition according to claim 1 or 2, wherein the composition additionally contains a reducing agent the electrode potential of which is more positive than that of ascorbic acid but more negative than that of uric acid.

4. The oxidation hair dye composition according to claim 3, wherein the composition contains less than 0.5% by weight of the reducing agent.

5. The oxidation hair dye composition according to any of claims 1 to 4, wherein the composition contains a pH adjusting agent selected from the group consisting of potassium hydroxide, monoethanolamine and a mixture thereof.

6. The oxidation hair dye composition according to any of claims 1 to 5, wherein the composition contains at least 60% by weight of water.

7. The oxidation hair dye composition according to any of claims 1 to 6, wherein the composition contains uricase in a concentration of 100 to 250 I.U./g.

8. The oxidation hair dye composition according to any of claims 1 to 7, wherein the composition is in the form of an aerosol.

9. A method of dyeing hair, comprising treating the hair with a one-pack type oxidation hair dye composition according to any of claims 1 to 8.

10. A method for stabilizing uricase in a one-pack type oxidation hair dye composition comprising uricase and an oxidation dye which comprises adding uric acid and optionally a reducing agent the electrode potential of which is more positive than that of ascorbic acid but more negative than that of uric acid to said composition and adjusting pH of said composition to 6.7 to 9.5.
